# EUROPEAN PATENT SPECIFICATION

(11) **EP 1 123 069 B1**
(45) Date of publication and mention of the grant of the patent: **06.02.2008**
(21) Application number: 98965636.8
(22) Date of filing: 20.10.1998
(51) Int. Cl.: A61F 2/44

(54) **STRAIN REGULATING FUSION CAGE FOR SPINAL FUSION SURGERY**
SPANNUNGSREGULIERENDER FUSIONSKÄFIG FÜR WIRBELSÄULENFUSIONSCHIRURGIE
CAGE D'ARTHRODESE SPINALE REGULATRICE DE CONTRAINTES POUR CHIRURGIE DE SOUDURE

(43) Date of publication of application: 16.08.2001
(73) Proprietor: Synthes GmbH, 4436 Oberdorf (CH)
(72) Inventor: BRESINA, Stephen, J., CH-7260 Davos (CH); TAGWERKER, Konrad, CH-4053 Basel (CH); SCHÄR, Manuel, CH-4132 Muttenz (CH)
(74) Representative: Lusuardi, Werther
(86) International application number: PCT/EP1998/006621
(87) International publication number: WO 2000/023014

(56) References cited:
- EP-A- 0 538 183
- WO-A-98/14142
- US-A- 5 423 817
- US-A- 5 749 916

## Description

The present invention relates to an intervertebral fusion cage according to the definition of claim 1.

Cage type implants are used for spinal fusion surgeries. The cage provides support until the graft material ossifies and fuses the two adjacent vertebral body endplates together. The sooner the ossification occurs and fusion is completed, the better for the patient.

From prior art several intervertebral implants are known. WO 98/09586 WEBB discloses a hollow cylindrical intervertebral implant made essentially of a ceramic material presenting a maximum porosity of 30 vol.% and which pores are filled with air. This known intervertebral implant is designed to bear the different loadings onto the vertebral column and provides a support at its end plates large enough to prevent these end plates from sinking into the adjacent vertebral bodies.

Another intervertebral implant is known from WO 97/15248 COTTLE. It consists of a frame like cage enclosing a space. This known cage is substantially wedge-shaped with top and bottom surfaces diverging towards the front wall, providing the advantage that, owing to the large bone bearing area of the top and bottom surfaces, the implant is prevented from sinking into the end plates of the body of the vertebra.

All these known existing cages, even with their intricate cutout patterns are quite stiff and shield the graft from stress and strain as well.

The features A), B) and E) of present claim 1 are known from the document EP-A-0 538 183.

Still another intervertebral implant is known from WO 97/15247 KNOTHE. It consists of a flattened shaped hollow element. The upper and lower bone-contact surfaces can be compressed elastically towards the inner chamber of the element in such a way that the maximum distance between the upper and lower bone contact surfaces can be reduced by 0,5 - 5,0 mm.

With these prior art hollow cages, the graft introduced into the cage endures strains that are proportional to the load.

Another intervertebral implant is known from EP-A 0 716 841 RATRON. The disclosed prosthesis provides an elastically deformable body having a spring rate k₁ until an upper aperture within the prosthesis closes under a certain load. Once this upper aperture is closed a spring rate k₂ will be reached which is different from the spring rate k₁ then causing the adjacent vertebral bodies to endure a higher load. This known intervertebral implant does not provide one or more cavities in the normal direction wherein graft material could be introduced and ossify fusing the two adjacent vertebral body endplates together. The different spring rates allow the prothesis to increase in stiffness as the end of the flexion/extension range of motion is reached.

It has been found that bone remodelling is controlled by peak strain, and that just a few cycles per day of strain above a certain level (1000 µε) is enough to maintain bone. Strains above 1000 µε and up to 5% or 50'000 µε proportionally increase new bone formation. If a fusion cage could allow the graft material to be exposed to these strain levels, the graft would be able to mineralize much sooner.

The strain ε is thereby defined as ε = δL/L whereby δL is the deformation of the body in the direction of the axis where the load is applied and L is the height or length of the unloaded body in the direction of the axis where the load will be applied.

One additional problem with standard cage designs including the last mentioned is that the strain applied to the graft will be different for different patients. A small patient will load the cage less than a large patient. If a patient is experiencing pain, he will load it much less than someone who feels good.

And only above a certain load level will the optimal strain level be reached. So the strain applied to the graft may never be appropriate for promoting bone formation. The known cages are stiff and the load required to produce a strain > 1000 µε can be high.

Here, the invention will offer remedy. The invention bases on the task to allow ideal strain levels to be attained in the enclosed graft material under minimal loads, while at the same time, protecting the graft from high strains which can lead to mechanical failure of the graft. The intervertebral cage is designed to be very flexible under small axial loads. Once the required strain level is reached, contact between the upper and lower portions of the cage significantly increases the stiffness of the device. Higher loads will only create very small additional strain. This allows a relatively consistent strain to be applied to the graft material irregardless of the applied physiological load.

In one preferred embodiment of the intervertebral cage according to the invention the cage is designed such that it permits the cage to be very compliant in the vertical direction until a certain displacement is reached. This displacement can be designed into the implant to allow the graft to be exposed to the desired level of strain of 1000 to 50'000 µε preferably of 3000 to 10'000 µε.

once this displacement has been reached, contact between the upper and lower portions of the cage is made and then the cage becomes very stiff, permitting only very small amounts of additional strain for increased loads. This would allow identical strains to be placed on the graft irregardless of the applied load, e.g. 200 N or 1000 N.

The strain regulating fusion cage according to this preferred embodiment comprises a prismatic cage with an upper and a lower bone contact surface transverse to its longitudinal axis, a central cavity that extends between the upper and the lower contact surface for receiving bone graft material and a plurality of slots that perforate the circumferential sidewall transversely to the longitudinal axis. The slots are staggeredly arranged such that at each of two different heights two of an entity of four slots are provided whereby the slots at each height cover opposite sectors of the circumferential sidewall and are arranged at the two different heights such that the angular sum of all the sectors amounts to more than 360° and the slots at the two different heights partially overlap one another. Furthermore, the slots have a minimal width that upon compressing the body along the central axis up to the desired level of strain the slots close elastically at their minimal width and significantly increase the stiffness of the cage. This minimal width depends on the height of the implant and on the desired strain level.

In another preferred embodiment of the strain regulating fusion cage according to the invention the height of the cage along the longitudinal axis amounts to 6 mm and the slots in their unloaded state have a width of 0,018 mm measured in the direction of the longitudinal axis. When the slots of the strain regulating fusion cage having these dimensions are closed under the required load the resulting strain level amounts to 3000 µε.

In yet a further embodiment of the strain regulating fusion cage according to the invention the height of the cage along the central axis amounts to 15 mm and the slots in their unloaded state have a width of 0,15 mm the resulting strain level amounts to 10'000 µε when the slots close under the load applied.

The invention and further embodiments of the invention are discussed in more detail in the following section by means of the partial schematic drawings.

The figures show:
Fig. 1 a lateral view of a section of the vertebral column with an implanted strain regulating fusion cage according to one embodiment of the invention in a lumbar application;
Fig. 2 a schematic representation of a strain regulation fusion cage according to the invention;
Fig. 3 a cross section of a schematic representation of a strain regulation fusion cage according to the invention shown in Fig. 2;
Fig. 4 another cross section of a schematic representation of a strain regulation fusion cage according to the invention shown in Fig. 2;
Fig. 5 a perspective view of a strain regulating fusion cage according to one embodiment of the invention;
Fig. 6 another perspective view of a strain regulating fusion cage according to the embodiment of the invention shown in Fig. 5;
Fig. 7 a lateral view of a strain regulating fusion cage according to the embodiment of the invention shown in Fig. 5;
Fig. 8 a lateral view of a strain regulating fusion cage according to the embodiment of the invention shown in Fig. 5 whereby the lower slots are closed at their minimal widths;
Fig. 9 a lateral view of a strain regulating fusion cage according to the embodiment of the invention shown in Fig. 5 whereby the lower and upper slots are closed at their minimal widths;
Fig. 10 a diagram representing the variable spring rate dependent of the strain applied to a strain regulating fusion cage according to the embodiment of the invention shown in Fig. 5; and
Fig. 11 a lateral view of a section of the vertebral column having a strain regulating fusion cage according to one embodiment of the invention implanted in an intervertebral space;

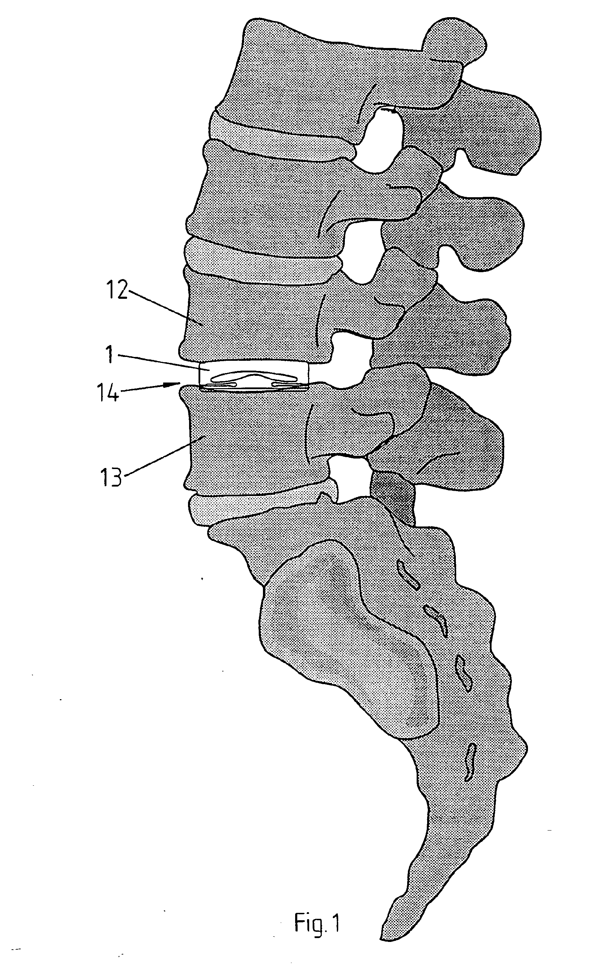

Fig. 1 shows a lumbar application of the strain regulating fusion cage 1 according to one embodiment of the invention implanted in an intervertebral space 14 between two vertebral bodies 12;13.

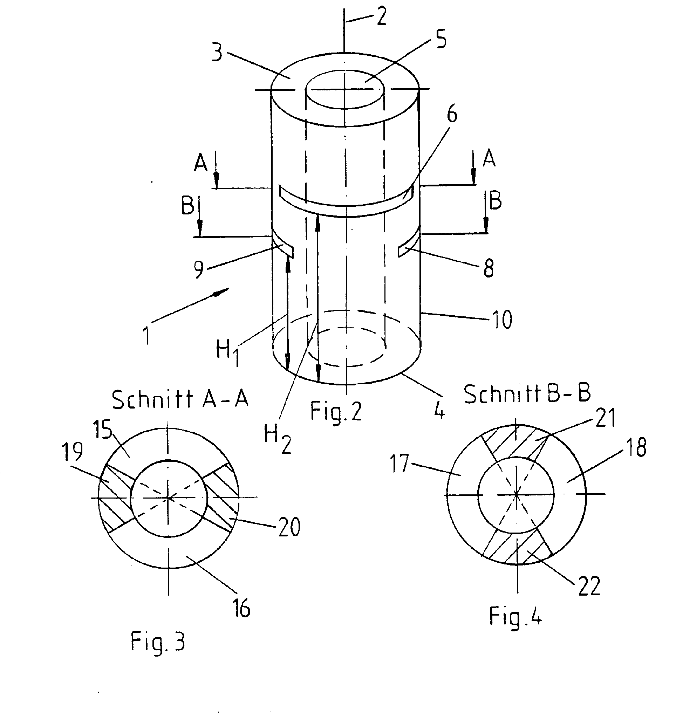

In Fig. 2 a schematic representation of a strain regulation fusion cage according to the invention is shown. The cage 1 consists of a hollow cylinder with a central axis 2, an upper contact surface 3, a lower contact surface 4 and a coaxial cavity 5 extending between the upper 3 and the lower contact surface 4. At a height H₁ two sectorial slots 8;9 perforate the circumferential sidewall 10 symmetrical to a first diameter and from diametrical opposite directions forming sectors 17;18 as shown in Fig. 4. Another two sectorial slots 6;7 (slot 7 not shown in the draft) perforate the circumferential sidewall 10 at a height H₂ that is closer to the upper contact surface 3 as the height H₁. These slots 8;9 arranged at the upper height H₂ also perforate the circumferential sidewall 10 symmetrical to a second diameter and from diametrical opposite directions forming sectors 15;16 as shown in Fig. 3. The slots 6;7 at the upper height H₂ arranged symmetrically to the second diameter are staggeredly arranged to the slots 8;9 at the lower height H₁ (fig. 4) arranged symmetrically to the first diameter whereby the first diameter is orthogonal to the second diameter. Furthermore, the slots 6;7 covering the sectors 15;16 at the upper height H₂ partially overlap the slots 8;9 covering the sectors 17;18 at the lower height H₁. Such the struts 19;20;21;22 remaining between the slots 6;7;8;9 at the circumferential sidewall 10 may be elastically compressed by what means the cage 1 is compressed.

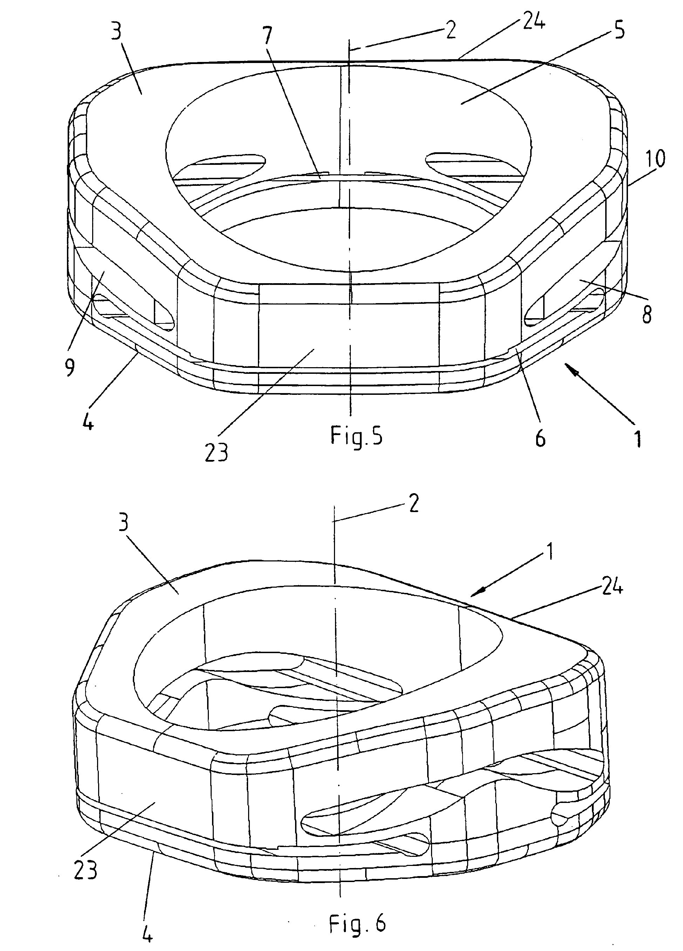

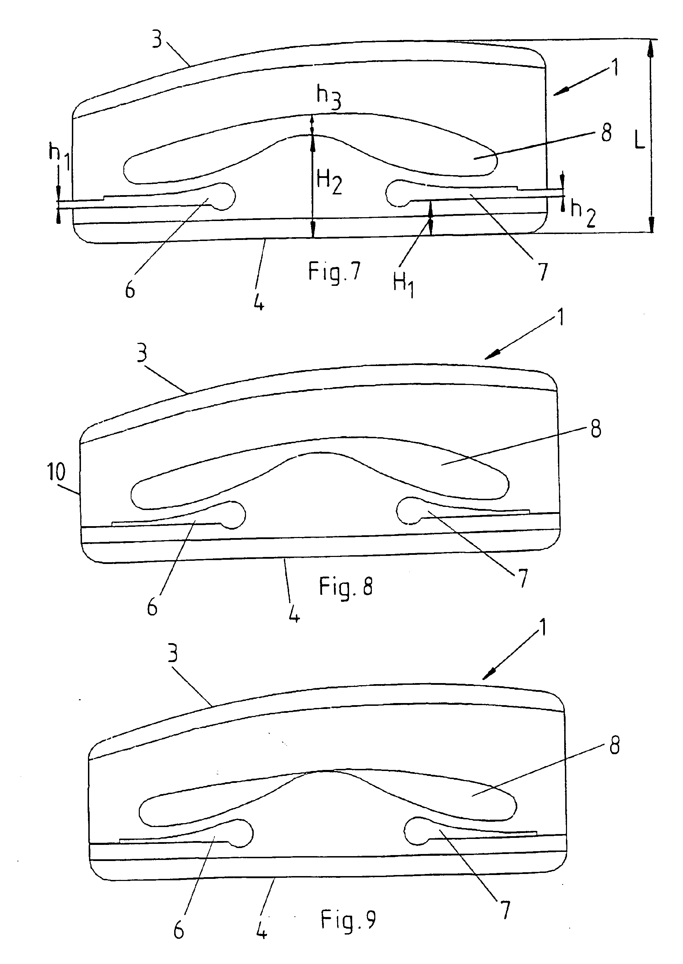

Fig. 5 and 6 show the preferred embodiment of the strain regulation fusion cage 1 according to the invention. The cage 1 has a prism like exterior shape with an upper contact surface 3, a lower contact surface 4, a longitudinal axis 2 and an oval hole 5 coaxial to the longitudinal axis 2 and extending between the upper 3 and the lower contact surface 4. The cross section perpendicular to the longitudinal axis 2 shows an exterior circumference of the cage 1 that has the shape of an irregular polygon. The lower contact surface 4 is even and extending perpendicular to the longitudinal axis 2. Transverse to the front side 23 of the cage 1 the upper contact surface 3 is convexly shaped and converges towards the lower contact surface 4 at the front side 23 and the back side 24. Parallel to the front side 23 of the cage 1 the upper contact surface 3 is not curved so that the cage 1 provides a wedgelike shape. The slots 6;7;8;9 perforate the circumferential sidewall 10 of the cage 1 at two planes perpendicular to the longitudinal axis 2 whereby the planes are situated at two different heights H₁;H₂ above the lower contact surface 4. Each plane contains two slots 6;7;8;9 that are situated diametrically opposite within the circumferential sidewall 10. The two slots 6;7 in the plane with the height H₁ which is closer to the lower contact surface 4 (fig. 7) are running parallel to the front side 23 of the cage 1 while the other two slots 8;9 in the plane with the height H₂ which is closer to the upper contact surface 3 (fig. 7) are running orthogonal to the front side 23 of the cage 1 such that the slots 6;7;8;9 are staggeredly configured at two different heights H₁;H₂ and each slot 6;7;8;9 covers another sector of the circumferential sidewall 10. Furthermore, the slots 6;7 in the plane closer to the lower contact surface 4 are only partially parallel shaped whereby these parallel sections provide the minimal width h₁;h₂ (fig. 7) of the slots 6;7. At the nonparallel sections the slots 6;7 provide a curved shape. The slots 8;9 in the plane with the greater height H₂ are curvedly shaped whereby the curves form a small almost linelike area with the minimal width h₃;h₄ of the slots 7;8.

Fig. 8 represents the strain regulating fusion cage 1 shown in fig. 5,6 and 7 whereby the cage 1 is compressed so far that the slots 6;7 lying in the plane closer to the lower contact surface 4 are closed at their sections with the minimal widths h₁;h₂.

In fig. 9 the strain regulation fusion cage 1 shown in fig. 5,6,7 and 8 is loaded such that the cage 1 is compressed so far that the slots 6;7 lying in the plane closer to the lower contact surface 4 and the slots 8;9 lying in the plane closer to the upper contact surface 3 are closed at their sections with the minimal widths h₁;h₂;h₃;h₄.

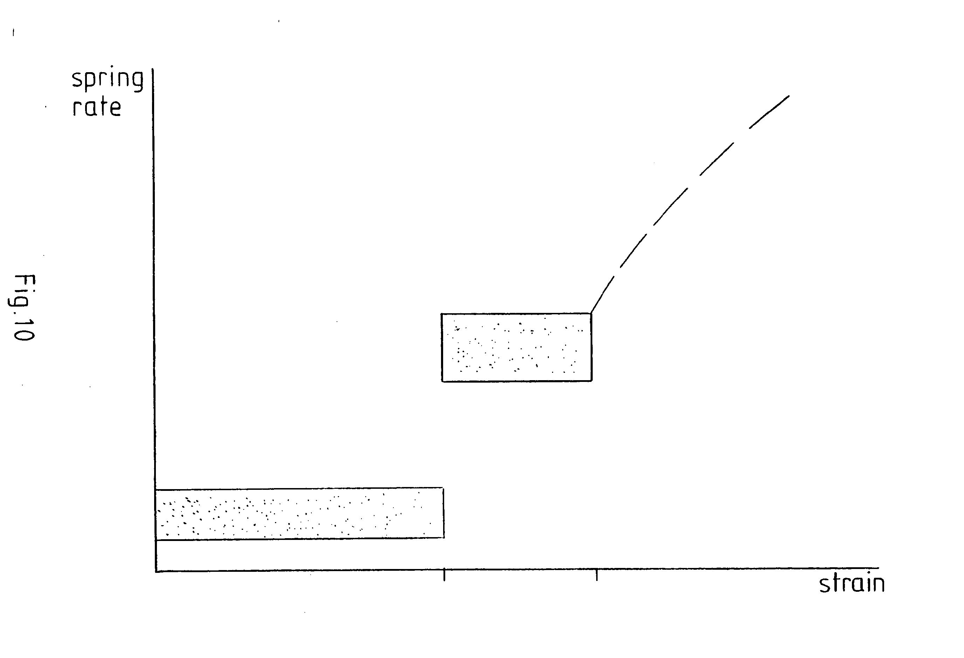

Fig. 10 illustrates the spring rate of the cage 1 wherein the cage 1 coaxially provides a spring rate c1 upon compression until the first set of the slots 6;7 closes at their minimal widths h₁;h₂ and upon further compression provides a spring rate c₂ amounting 1 and 5 times as much as c₁ until the second set of slots 8;9 closes at their minimal widths h₃;h₄ causing a further increase of the stiffness of the cage 1 with an unknown gradient of the spring rate.

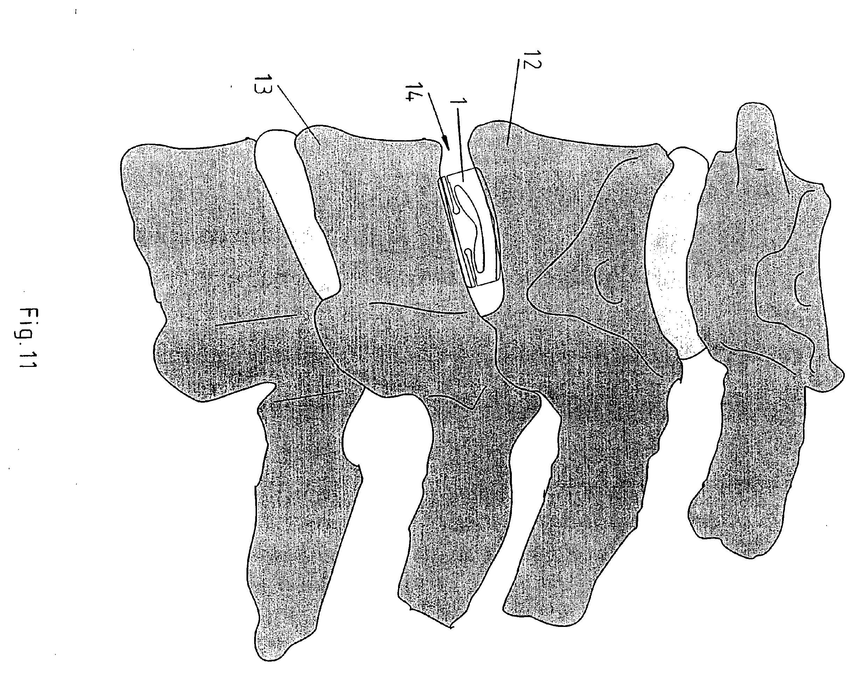

Fig. 11 shows the strain regulating fusion cage 1 according to one embodiment of the invention implanted in an intervertebral space 14 between two vertebral bodies 12;13.

## Claims

1. Intervertebral fusion cage comprising a prismatic, conical or cylindrical cage (1) having
A) a central axis (2);
B) an upper and a lower contact surface (3;4) transverse to said central axis (2), whereby said upper and lower contact surfaces (3;4) are destined for contacting two adjacent vertebral bodies (12;13) upon implanting the body (1) into the intervertebral space (14);
C) a central cavity (5) for receiving bone graft material and extending between said lower contact surface (4) and said upper contact surface (3) through said body (1) defining a circumferential sidewall (10) arranged coaxially to said central axis (2); and
D) a plurality of sectorial slots (6;7;8;9) perforating the circumferential sidewall (10) transversely to the central axis (2),whereby
E) said slots (6;7;8;9) have a minimal width (h1;h2;h3;h4) that upon compressing of the body (1) along the central axis (2) said slots (6;7;8;9) close elastically at the minimal widths (h1;h2;h3;h4) thereby increasing the stiffness of the cage (1) upon further compression.

2. Intervertebral fusion cage according to claim 1, **characterized in that** upon compressing of the body (1) along the central axis (2) to a strain level of between 1000 µε and 50'000 µε said slots (6;7;8;9) close elastically at the minimal widths (h₁;h₂;h₃;h₄) thereby increasing the stiffness of the cage (1) upon further compression.

3. Intervertebral fusion cage according to claim 2, **characterized in that** upon compressing of the body (1) along the central axis (2) to a strain level of between 3000 µε and 10'000 µε said slots (6;7;8;9) close elastically at the minimal widths (h₁;h₂;h₃;h₄) thereby increasing the stiffness of the cage (1) upon further compression.

4. Intervertebral fusion cage according to one of the claims 1 to 3, **characterized in that** upon compression the cage (1) coaxially provides a spring rate c₁ until the slots (6;7;8;9) close at their minimal widths (h₁;h₂;h₃;h₄) and upon further compression provides a spring rate c₂ amounting between 10 and 100 times as much as c₁.

5. Intervertebral fusion cage according to one of the claims 1 to 4, **characterized in that** upon compression the cage (1) coaxially provides a spring rate c₁ until a first set of the slots (6;7;8;9) closes at their minimal widths (h₁;h₂;h₃;h₄) and upon further compression provides a spring rate c₂ amounting between 1,0 and 5 times as much as c₁ until a second set of the slots (6;7;8;9) closes at their minimal widths (h₁;h₂;h₃; h₄) causing a further increase of the stiffness of the cage (1).

6. Intervertebral fusion cage according to one of the claims 1 to 5, **characterized in that** said slots (6;7;8;9) perforate the circumferential sidewall (10) at at least two different heights (H₁;H₂) above the lower contact surface (4).

7. Intervertebral fusion cage according to one of the claims 1 to 6, **characterized in that** said slots (6;7;8;9) are staggeredly configured at at least two different heights (H₁;H₂).

8. Intervertebral fusion cage according to one of the claims 1 to 7, **characterized in that** said slots (6;7;8;9) are staggeredly configured at two different heights (H₁;H₂).

9. Intervertebral fusion cage according to one of the claims 1 to 8, **characterized in that** each slot (6;7;8;9) covers another sector (15;16;17;18) of said circumferential sidewall (10) such that the angular sum of all the sectors amounts to at least 360°.

10. Intervertebral fusion cage according to claim 9, **characterized in that** the sectors (15;16;17;18) partially overlap one another.

11. Intervertebral fusion cage according to one of the claims 1 to 10, **characterized in that** said slots (6;7;8;9) provide a width (h₁;h₂;h₃;h₄) that once the body (1) is compressed along the central axis (2) to a strain level of between 1000 µε and 50'000 µε the slots (6;7;8;9) elastically close at the minimal widths (h₁;h₂;h₃;h₄).

12. Intervertebral fusion cage according to claim 11, **characterized in that** said slots (6;7;8;9) provide a width (h₁;h₂;h₃;h₄) that once the body (1) is compressed along the central axis (2) to a strain level of between 3000 µε and 10'000 µε the slots (6;7;8;9) elastically close at the minimal widths (h₁;h₂;h₃;h₄).

13. Intervertebral fusion cage according to one of the claims 1 to 12, **characterized in that** the slots (6;7;8;9) provide a minimal width (h₁;h₂;h₃;h₄) which amounts between 0,02 to 0,15 mm.

14. Intervertebral fusion cage according to one of the claims 1 to 13, **characterized in that** the cage (1) is symmetrical to a plane containing the central axis (2).

15. Intervertebral fusion cage according to one of the claims 1 to 14, **characterized in that** at each of two different heights (H₁;H₂) two of an entity of four slots (6;7;8;9) are provided.

16. Intervertebral fusion cage according to one of the claims 1 to 15, **characterized in that** at the height (H₁) which is closer to the lower contact surface (4) two slots (6;7) are provided at opposite sectors (15;16) of said circumferential sidewall (10).

17. Intervertebral fusion cage according to one of the claims 1 to 16, **characterized in that** at the height (H₂) which is closer to the upper contact surface (3) two slots (8;9) are provided at opposite sectors (17;18) of said circumferential sidewall (10).

18. Intervertebral fusion cage according to one of the claims 1 to 17, **characterized in that** each of said sectors encloses an angle ranging between 45° and 150°, preferably between 90° and 120°.

19. Intervertebral fusion cage according to one of the claims 1 to 18, **characterized in that** the volume of the cavity (5) amounts between 30% and 70%, preferably between 40% and 60% of the volume enclosed by the exterior surfaces of the cage (1).

20. Intervertebral fusion cage according to one of the claims 1 to 19, **characterized in that** the slots (6;7;8;9) provide a variable width in the unloaded state of the cage (1).

## Patentansprüche

1. Fusionskäfig für den Zwischenwirbelraum umfassend einen prismatischen, konischen oder zylindrischen Käfig (1) mit
A) einer Zentralachse (2);
B) einer oberen und einer unteren Kontaktfläche (3;4) quer zur genannten Zentralachse (2), wobei die genannte obere und untere Kontaktfläche (3;4) zur Anlage an zwei benachbarte Wirbelkörper (12;13) bei in den Zwischenwirbelraum (14) implantiertem Käfig (1) bestimmt sind;
C) einem zentralen Hohlraum (5) zur Aufnahme von Knochenpfropfenmaterial, welcher sich zwischen der genannten unteren Kontaktfläche (4) und der genannten oberen Kontaktfläche (3) durch den Käfig (1) erstreckt und eine koaxial zur genannten Zentralachse (2) angeordnete periphere Seitenwand (10) definiert; und
D) einer Anzahl sektorieller Schlitze (6;7;8;9), welche die periphere Seitenwand (10) quer zur Zentralachse (2) durchdringen, wobei
E) die genannten Schlitze (6;7;8;9) eine Minimalweite (h1;h2;h3;h4) haben, so dass sich bei einer Kompression des Käfigs (1) entlang der Zentralachse (2) die genannten Schlitze (6;7;8;9) bei der Minimalweite (h1;h2;h3;h4) elastisch schliessen und damit die Steifigkeit des Käfigs (1) bei einer weiteren Kompression erhöhen.

2. Fusionskäfig nach Anspruch 1, **dadurch gekennzeichnet, dass** sich die genannten Schlitze (6;7;8;9) bei einer Kompression des Käfigs (1) entlang der Zentralachse (2) bei der Minimalweite (h1;h2;h3;h4) bis zu einem Verformungsgrad zwischen 1000 µε und 50'000 µε elastisch schliessen und damit die Steifigkeit des Käfigs (1) bei einer weiteren Kompression erhöhen.

3. Fusionskäfig nach Anspruch 2, **dadurch gekennzeichnet, dass** sich die genannten Schlitze (6;7;8;9) bei einer Kompression des Käfigs (1) entlang der Zentralachse (2) bei der Minimalweite (h1;h2;h3;h4) bis zu einem Verformungsgrad zwischen 3000 µε und 10'000 µε elastisch schliessen und damit die Steifigkeit des Käfigs (1) bei einer weiteren Kompression erhöhen.

4. Fusionskäfig nach einem der Ansprüche 1 bis 3, **dadurch gekennzeichnet, dass** der Käfig (1) bei einer koaxialen Kompression eine Federrate c₁ aufweist bis sich die Schlitze (6;7;8;9) bei ihrer Minimalweite (h1;h2;h3;h4) schliessen und bei einer weiteren Kompression eine Federrate c₂ aufweist, welche zwischen dem 10-fachen und dem 100-fachen von c₁ beträgt.

5. Fusionskäfig nach einem der Ansprüche 1 bis 4, **dadurch gekennzeichnet, dass** der Käfig (1) bei einer koaxialen Kompression eine Federrate c₁ aufweist bis sich ein erster Satz von Schlitzen (6;7;8;9) bei deren Minimalweite (h1;h2;h3;h4) schliesst und bei einer weiteren Kompression eine Federrate c₂ aufweist, welche zwischen dem 1,0-fachen und dem 5-fachen von c₁ beträgt bis sich ein zweiter Satz von Schlitzen (6;7;8;9) bei deren Minimalweite (h1;h2;h3;h4) schliesst und damit eine weitere Erhöhung der Steifigkeit des Käfigs (1) verursachen.

6. Fusionskäfig nach einem der Ansprüche 1 bis 5, **dadurch gekennzeichnet, dass** die genannten Schlitze (6;7;8;9) die periphere Seitenwand (10) bei mindestens zwei unterschiedlichen Höhen (H₁;H₂) über der unteren Kontaktfläche (4) durchdringen.

7. Fusionskäfig nach einem der Ansprüche 1 bis 6, **dadurch gekennzeichnet, dass** die genannten Schlitze (6;7;8;9) versetzt bei mindestens zwei unterschiedlichen Höhen (H₁;H₂) angeordnet sind.

8. Fusionskäfig nach einem der Ansprüche 1 bis 7, **dadurch gekennzeichnet, dass** die genannten Schlitze (6;7;8;9) versetzt bei zwei unterschiedlichen Höhen (H₁;H₂) angeordnet sind.

9. Fusionskäfig nach einem der Ansprüche 1 bis 8, **dadurch gekennzeichnet, dass** sich jeder Schlitz (6;7;8;9) über einen anderen Sektor (15;16;17;18) der genannten peripheren Seitenwand (10) erstreckt, so dass die Summe der Winkel aller Sektoren mindestens 360° beträgt.

10. Fusionskäfig nach Anspruch 9, **dadurch gekennzeichnet, dass** sich die Sektoren (15;16;17;18) teilweise gegenseitig überlappen.

11. Fusionskäfig nach einem der Ansprüche 1 bis 10, **dadurch gekennzeichnet, dass** die genannten Schlitze (6;7;8;9) eine Weite (h1;h2;h3;h4) aufweisen, so dass sich die Schlitze (6;7;8;9) bei einer Kompression des Käfigs (1) entlang der Zentralachse (2) bis zu einem Verformungsgrad zwischen 1000 µε und 50'000 µε bei der Minimalweite (h1;h2;h3;h4) elastisch schliessen.

12. Fusionskäfig nach Anspruch 11, **dadurch gekennzeichnet, dass** die genannten Schlitze (6;7;8;9) eine Weite (h1;h2;h3;h4) aufweisen, so dass sich die Schlitze (6;7;8;9) bei einer Kompression des Käfigs (1) entlang der Zentralachse (2) bis zu einem Verformungsgrad zwischen 3000 µε und 10'000 µε bei der Minimalweite (h1;h2;h3;h4) elastisch schliessen.

13. Fusionskäfig nach einem der Ansprüche 1 bis 12, **dadurch gekennzeichnet, dass** die Schlitze (6;7;8;9) eine Minimalweite (h1;h2;h3;h4) aufweisen, welche zwischen 0,02 bis 0,15 mm beträgt.

14. Fusionskäfig nach einem der Ansprüche 1 bis 13, **dadurch gekennzeichnet, dass** der Käfig (1) zu einer die Zentralachse (2) enthaltenden Ebene symmetrisch ist.

15. Fusionskäfig nach einem der Ansprüche 1 bis 14, **dadurch gekennzeichnet, dass** an jeder von zwei unterschiedlichen Höhen (H₁;H₂) zwei aus einer Gesamtheit von vier Schlitzen (6;7;8;9) vorgesehen sind.

16. Fusionskäfig nach einem der Ansprüche 1 bis 15, **dadurch gekennzeichnet, dass** bei der Höhe (H₁), welche näher bei der unteren Kontaktfläche (4) ist, zwei Schlitze (6;7) in gegenüberliegenden Sektoren (15;16) der genannten peripheren Seitenwand (10) angeordnet sind.

17. Fusionskäfig nach einem der Ansprüche 1 bis 16, **dadurch gekennzeichnet, dass** bei der Höhe (H₂), welche näher bei der oberen Kontaktfläche (3) ist, zwei Schlitze (6;7) in gegenüberliegenden Sektoren (15;16) der genannten peripheren Seitenwand (10) angeordnet sind.

18. Fusionskäfig nach einem der Ansprüche 1 bis 17, **dadurch gekennzeichnet, dass** jeder der genannten Sektoren einen Winkel in einem Bereich zwischen 45° und 150°, vorzugsweise zwischen 90° und 120° einschliesst.

19. Fusionskäfig nach einem der Ansprüche 1 bis 18, **dadurch gekennzeichnet, dass** das Volumen des Hohlraums (5) zwischen 30% und 70%, vorzugsweise zwischen 40% und 60% des Volumens beträgt, welches durch Aussenfläche des Käfigs (1) eingeschlossen wird.

20. Fusionskäfig nach einem der Ansprüche 1 bis 19, **dadurch gekennzeichnet, dass** die Schlitze (6;7;8;9) im unbelasteten Zustand des Käfigs (1) eine variable Weite aufweisen.

## Revendications

1. Cage de fusion intervertébrale comprenant une cage prismatique, conique ou cylindrique (1) comprenant
A) un axe central (2) ;
B) des surfaces de contact supérieure et inférieure (3 ; 4) transversales audit axe central (2), dans laquelle lesdites surfaces de contact supérieure et inférieure (3 ; 4) sont destinées à mettre en contact deux corps vertébraux adjacents (12 ; 13) lors de l'implantation du corps (1) dans l'espace intervertébral (14) ;
C) une cavité centrale (5) destinée à recevoir une matière de greffon osseux et s'étendant entre ladite surface de contact inférieure (4) et ladite surface de contact supérieure (3) à travers ledit corps (1) définissant une paroi latérale circonférentielle (10) disposée coaxialement audit axe central (2) ; et
D) une pluralité de fentes sectorielles (6 ; 7 ; 8 ; 9) perforant la paroi latérale circonférentielle (10) transversalement à l'axe central (2), dans laquelle
E) lesdites fentes (6 ; 7 ; 8 ; 9) ont une largeur minimale (h₁ ; h₂ ; h₃ ; h₄) et, lors de la compression du corps (1) suivant l'axe central (2), lesdites fentes (6 ; 7 ; 8 ; 9) se ferment de manière élastique jusqu'aux largeurs minimales (h₁ ; h₂ ; h₃ ; h₄) augmentant alors la rigidité de la cage (1) lors d'une compression supplémentaire.

2. Cage de fusion intervertébrale selon la revendication 1, **caractérisée en ce que**, lors de la compression du corps (1) suivant l'axe central (2) jusqu'à un niveau de déformation compris entre 1 000 µε et 50 000 µε, lesdites fentes (6 ; 7 ; 8 ; 9) se ferment de manière élastique jusqu'aux largeurs minimales (h₁ ; h₂; h₃; h₄) augmentant alors la rigidité de la cage (1) lors d'une compression supplémentaire.

3. Cage de fusion intervertébrale selon la revendication 2, **caractérisée en ce que**, lors de la compression du corps (1) suivant l'axe central (2) jusqu'à un niveau de déformation compris entre 3 000 µε et 10 000 µε, lesdites fentes (6 ; 7 ; 8 ; 9) se ferment de manière élastique jusqu'aux largeurs minimales (h₁ ; h₂; h₃; h₄) augmentant alors la rigidité de la cage (1) lors d'une compression supplémentaire.

4. Cage de fusion intervertébrale selon l'une des revendications 1 à 3, **caractérisée en ce que**, lors de sa compression, la cage (1) présente coaxialement une constante de rappel c₁ jusqu'à ce que les fentes (6 ; 7 ; 8 ; 9) se ferment à leurs largeurs minimales (h₁ ; h₂ ; h₃ ; h₄) et, lors d'une compression supplémentaire, présente une constante de rappel c₂ atteignant une valeur comprise entre 10 et 100 fois celle de c₁.

5. Cage de fusion intervertébrale selon l'une des revendications 1 à 4, **caractérisée en ce que**, lors de sa compression, la cage (1) présente coaxialement une constante de rappel c₁ jusqu'à ce qu'un premier jeu de fentes (6 ; 7 ; 8 ; 9) se ferment à leurs largeurs minimales (h₁ ; h₂ ; h₃ ; h₄) et, lors d'une compression supplémentaire, présente une constante de rappel c₂ atteignant une valeur comprise entre 1,0 et 5 fois celle de c₁ jusqu'à ce qu'un deuxième jeu de fentes (6 ; 7; 8 ; 9) se ferment à leurs largeurs minimales (h₁ ; h₂ ; h₃ ; h₄) augmentant davantage la rigidité de la cage (1).

6. Cage de fusion intervertébrale selon l'une des revendications 1 à 5, **caractérisée en ce que** lesdites fentes (6 ; 7 ; 8 ; 9) perforent la paroi latérale circonférentielle (10) à au moins deux hauteurs différentes (H₁ ; H₂) au-dessus de la surface de contact inférieure (4).

7. Cage de fusion intervertébrale selon l'une des revendications 1 à 6, **caractérisée en ce que** lesdites fentes (6 ; 7 ; 8 ; 9) sont disposées de façon décalée à au moins deux hauteurs différentes (H₁ ; H₂).

8. Cage de fusion intervertébrale selon l'une des revendications 1 à 7, **caractérisée en ce que** lesdites fentes (6 ; 7 ; 8 ; 9) sont disposées de façon décalée à deux hauteurs différentes (H₁ ; H₂).

9. Cage de fusion intervertébrale selon l'une des revendications 1 à 8, **caractérisée en ce que** chaque fente (6 ; 7 ; 8 ; 9) couvre un secteur différent (15 ; 16 ; 17 ; 18) de ladite paroi latérale circonférentielle (10) de sorte que la somme des angles de tous les secteurs s'élève au moins à 360°.

10. Cage de fusion intervertébrale selon la revendication 9, **caractérisée en ce que** les secteurs (15 ; 16 ; 17 ; 18) se chevauchent partiellement.

11. Cage de fusion intervertébrale selon l'une des revendications 1 à 10, **caractérisée en ce que** lesdites fentes (6 ; 7 ; 8 ; 9) présentent une largeur (h₁ ; h₂ ; h₃ ; h₄) et, une fois que le corps (1) est comprimé suivant l'axe central (2) jusqu'à un niveau de déformation compris entre 1 000 µε et 50 000 µε, les fentes (6 ; 7 ; 8 ; 9) se ferment de manière élastique jusqu'aux largeurs minimales (h₁ ; h₂ ; h₃ ; h₄).

12. Cage de fusion intervertébrale selon la revendication 11, **caractérisée en ce que** lesdites fentes (6 ; 7 ; 8 ; 9) présentent une largeur (h₁ ; h₂ ; h₃ ; h₄) et, une fois que le corps (1) est comprimé suivant l'axe central (2) jusqu'à un niveau de déformation compris entre 3 000 µε et 10 000 µε, les fentes (6 ; 7 ; 8 ; 9) se ferment de manière élastique jusqu'aux largeurs minimales (h₁ ; h₂ ; h3 ; h₄).

13. Cage de fusion intervertébrale selon l'une des revendications 1 à 2, **caractérisée en ce que** les fentes (6 ; 7 ; 8 ; 9) présentent une largeur minimale (h₁ ; h₂ ; h₃ ; h₄) qui est comprise entre 0,02 mm et 0,15 mm.

14. Cage de fusion intervertébrale selon l'une des revendications 1 à 13, **caractérisée en ce que** la cage (1) est symétrique à un plan contenant l'axe central (2).

15. Cage de fusion intervertébrale selon l'une des revendications 1 à 14, **caractérisée en ce que**, à chacune des deux différentes hauteurs (H₁ ; H₂), deux entités des quatre fentes (6 ; 7 ; 8 ; 9) sont fournies.

16. Cage de fusion intervertébrale selon l'une des revendications 1 à 15, **caractérisée en ce que**, à la hauteur (H₁) qui est la plus proche de la surface de contact inférieure (4), deux fentes (6 ; 7) sont formées au niveau de secteurs opposés (15 ; 16) de ladite paroi latérale circonférentielle (10).

17. Cage de fusion intervertébrale selon l'une des revendications 1 à 16, **caractérisée en ce que**, à la hauteur (H₂) qui est la plus proche de la surface de contact supérieure (3), deux fentes (8 ; 9) sont formées au niveau de secteurs opposés (17 ; 18) de ladite paroi latérale circonférentielle (10).

18. Cage de fusion intervertébrale selon l'une des revendications 1 à 17, **caractérisée en ce que** chacun desdits secteurs forme un angle compris entre 45° et 150°, de préférence entre 90° et 120°.

19. Cage de fusion intervertébrale selon l'une des revendications 1 à 18, **caractérisée en ce que** le volume de la cavité (5) est compris entre 30% et 70%, de préférence entre 40% et 60% du volume formé par les surfaces externes de la cage (1).

20. Cage de fusion intervertébrale selon l'une des revendications 1 à 19, **caractérisée en ce que** les fentes (6 ; 7 ; 8 ; 9) présentent une largeur variable dans l'état déchargé de la cage (1).
